# EUROPEAN PATENT APPLICATION

(11) **EP 1 806 052 A1**
(43) Date of publication of application: **11.07.2007**
(21) Application number: 07000078.1
(22) Date of filing: 03.01.2007
(51) Int. Cl.: A01K 67/027, G01N 33/50

(54) **Method for producing model animal of vascular wall lesion**

(30) Priority: 05.01.2006 JP 2006000526
(71) Applicant: Fujifilm Corporation, Minato-ku Tokyo (JP)
(72) Inventor: Aikawa, Kazuhiro, Ashigarakami-gun Kanagawa 258-8577 (JP)
(74) Representative: HOFFMANN EITLE

(57) **Abstract**

An object to be achieved by the present invention is to provide a method for producing a model animal of vascular wall lesion. The present invention provides a method for producing a non-human model animal of vascular wall lesion, which comprises invading a part of an artery of a non-human animal from outside the blood vessel and producing an animal having a lesion at the invaded vascular wall.

## Description

### Technical Field

The present invention relates to a method for producing a model animal of vascular wall lesion. More specifically, the present invention relates to a method for producing a model animal of vascular wall lesion, which comprises causing the development of a vascular wall lesion by invasion from outside the blood vessel. Furthermore, the present invention relates to a method for evaluating the effects of a drug using the above model animal of vascular lesion.

### Background Art

In the modern society and particularly in the advanced country's society, chances of having high calorie and high fat meals are increasing. Accordingly, the death toll from ischemic diseases (e.g., heart diseases such as cardiac infarction and angina pectoris, and cerebrovascular diseases such as cerebral infarction and cerebral hemorrhage) caused by arterial sclerosis is increasing. It is required to diagnose the symptoms of such ischemic diseases at earlier stages and properly treat the diseases. Furthermore, inner wall lesions of blood vessels due to hypertension are also not negligible as lesions that induce serious diseases. Such lesions are gradually formed over a long time period and suddenly lead to blood platelet aggregation, resulting in cerebral infarction or cardiac infarction. Therefore, it is important to diagnose these lesions of the inner walls of the blood vessels at earlier stages. However, there is currently no method for sufficiently diagnosing such lesions before the resulting attacks. One of reasons therefor is the absence of appropriate model animals of vascular wall lesion, with which tests are conducted for screening for candidate compounds of diagnostic agents.

Animals that enable rapid and low-cost production of pathological models of arterial sclerosis plaque are rats. Currently, the most general method for the formation of an arterial sclerosis plaque in a rat is a balloon catheter method (Phar. Res. 16, 420, 1993). According to this method, an arterial sclerosis plaque can be formed within a period as short as 10 days. However, this method involves insertion of a catheter into an artery, which requires a lot of skill. Furthermore, the efficiency of forming arterial sclerosis plaques is very poor, such that arterial sclerosis plaques are formed in only approximately 50% of animals subjected to the method.

Furthermore, JP Patent Publication (Kokai) No. 2002-58389 A discloses a method for producing a vascular disease pathological model animal, comprising the following steps of: (1) invading a part of an artery of an animal from outside the blood vessel; and (2) forming a lesion at the invaded blood vessel by feeding the animal with a feed supplemented with a chemical substance that induces a vascular disease. However, JP Patent Publication (Kokai) No. 2002-58389 A discloses nothing about model animals of vascular wall lesion.

When a vascular wall lesion is developed, blood platelets come into contact with subcutaneous tissues and adhere to subcutaneous tissue components such as collagen. Thus, reactions by which many blood platelets aggregate each other are caused, resulting in the release of various mediators. The effects of collagen and blood platelets are affected by shear stress. At a low shear rate, binding with a blood platelet membrane protein GPIa/IIa mainly takes place. At a high shear rate, binding with GPIb/IX via a von Willebrand factor (vWF) mainly takes place. vWFs produced in megakaryocytes are stored within blood platelet granules and are released when the blood platelets are activated. Some of secreted vWFs bind to extracellular matrices, and are present in intravascular subcutaneous tissues. Upon contact with subcutaneous tissues, vWFs change their conformation, so as to be able to bind to blood platelets.

Blood platelets are activated when they adhere to subendothelial tissues. GPIIa/IIIb of the integrin family is then activated, and fibrinogen binds to the activated GPIIa/IIIb, causing aggregation reactions with other blood platelets. When blood platelets bind to collagen, ADP is released from dense granules. In this manner, the aggregation effect of blood platelets is advanced. Plasma comes into contact with subcutaneous tissues, so as to initiate coagulation reactions, thereby producing thrombin. Even a trace amount of thrombin enhances blood platelet aggregation. Regarding thrombin and collagen, thrombospondin, fibronectin and the like are also released from blood platelets, in addition to ADP, thereby stabilizing agglomerates. In blood platelet membranes where adhesion or aggregation takes place, phospholipase C and phospholipase A2 are activated, arachidon is released from phospholipids, cytoplasmic calcium ion concentrations are elevated, and cytoskeletons are altered. Hence, release reactions are further promoted. Arachidonic acid is metabolized by cyclooxygenase to prostaglandin H2, and then thromboxane A2 is produced by thromboxane synthase. Thromboxane A2 binds to a 7-transmembrane receptor, so as to strongly promote blood platelet aggregation.

Vascular alterations or the formation of arterial sclerosis plaques, which are caused by hypertension, are accompanied by the development of the above described vascular wall lesions. It is considered that accumulation of a drug at such blood platelet aggregation sites is effective for treating and/or diagnosing vascular wall lesions and arterial sclerosis plaques.

However, it is still difficult to produce model animals of vascular wall lesion. Rats are often used for production of model animals of vascular wall lesion, because they enable rapid and low-cost production of such model animals. Balloon catheters are used in a manner similar to that in the above case of forming arterial sclerosis plaques. However, production of vascular wall lesion model is even more difficult than the case of producing arterial sclerosis plaques. It has heretofore been impossible to produce vascular wall lesions that are of equal severity among subject animals.

A method for producing vascular wall lesions in rats or rabbits is also known, which involves invasion of the blood vessel from the outside. Specifically, a method that involves exposing a carotid artery and repeatedly anchoring the exposed site with tweezers without rupturing the blood vessel (Circulation 21 123 1988), a method that involves pinching such site with a clip without completely blocking blood flow (Pharm. Res 12 (41) 1765, 1996), a method that involves instantaneously applying heat to such site with an electrically-heated wire (Pham. Res. 13 (42) 2387, 2001), and the like are known. However, these methods have drawbacks such that the success rate of producing vascular wall lesions is 20% or less in all the methods and that these methods require a large number of animals in experiments.

### Summary of the Invention

An object to be achieved by the present invention is to provide a method for producing a model animal of vascular wall lesion. In particular, an object to be achieved by the present invention is to provide a method for producing a model animal of vascular wall lesion for the purpose of efficiently causing the development of a vascular wall lesion by invasion from outside the blood vessel and evaluating the diagnostic and therapeutic effects of a drug on the vascular lesion site using the animal having the lesion.

As a result of intensive studies to achieve the above objects, the present inventors have discovered a method for producing a model animal of vascular wall lesion, which comprises ligating an artery of a rat or a mouse using a surgical suture and raising the rat or the mouse for 4 to 10 days, and which enables the formation of a vascular wall lesion at the ligated part with a high success rate (98% or more of treated animals).

Specifically, the present invention provides a method for producing a non-human model animal of vascular wall lesion, which comprises invading a part of an artery of a non-human animal from outside the blood vessel and producing an animal having a lesion at the invaded vascular wall.

Preferably, a part of an artery is invaded from outside the blood vessel by ligation of the blood vessel.

Preferably, a part of an artery is invaded from outside the blood vessel by ligation of the blood vessel that enables ensuring a 50% to 80% blood flow.

Preferably, ligation is performed at 2 or more sites at 5-mm to 50-mm intervals.

Preferably, the animal is a dog, a miniature pig, a rabbit, a rat, a guinea pig, or a mouse.

Preferably, the site of a vascular wall lesion is a carotid artery.

Another aspect of the present invention provides a method for evaluating a test substance, which comprises administering a test substance to a non-human model animal of vascular wall lesion which is produced by the above method of the present invention, and evaluating the effects of the test substance on the vascular wall lesion.

### Best Mode for Carrying out the Invention

The present invention is described in detail below.

The method of the present invention is characterized in that a part of an artery of a non-human animal is invaded from outside the blood vessel and then an animal having a lesion at the invaded vascular wall is produced in order to produce a non-human model animal of vascular wall lesion.

As clearly shown in the results of the examples of the description, the percentage of rats that formed vascular wall lesions in the case of the arterial ligation method according to the present invention was 98%, whereas the same in the case of the balloon catheter method was 48%. That is, the probability of producing pathological models in the case of the arterial ligation method was improved so that it became twice or more than that in the case of the balloon catheter method. Moreover, whereas the raising period in the case of the arterial ligation method was 6 days, the same in the case of the balloon catheter method was 10 days; the raising period in the case of the arterial ligation method of the present invention was significantly shorter than that of the balloon catheter method, by 4 days. Therefore, compared with the conventional balloon catheter method, the arterial ligation method of the present invention realizes higher success rate of forming vascular wall lesions and shorter period of raising rats.

The types of the non-human animal to be used in the present invention are not particularly limited, as long as the animals are non-human animals. In general, birds and the like may also be used, in addition to mammals that are used as experimental animals. More specifically, guinea pigs, dogs, miniature pigs, monkeys, rabbits, rats, mice, flying squirrels, chickens, pigeons, and the like are preferably used. More preferable animals are animals selected from the group consisting of dogs, miniature pigs, rabbits, rats, and mice. Of these, rats or mice are further preferable, and rats are particularly preferable. Examples of rat types include lines such as Wistar, SD, Fisher, LE, and F-344. Preferably, SD rats can be used. Examples of mouse types include lines such as ICR, Bulb/cA, Nu/nu, B10, C57 brown, AKR, DBA, and C3H. Preferably, ICR mice can be used.

The types of arteries to be ligated are not particularly limited and are preferably carotid arteries. In the case of small animals such as rats, aortas, atrial arteries, hepatic arteries, descending arteries, and the like can be used. In the case of large animals, arteries of extremities and the like can also be used.

Methods for exposing arteries are not particularly limited. In general, arteries can be exposed aseptically by a technique such as general laparotomy under anesthetic.

Methods for invading an exposed artery from outside are not particularly limited. In general, means for maintaining a predetermined physical stress load on an artery can be appropriately selected. For example, a method for ligating arteries using surgical sutures can be suitably used. Ligation is desirably performed so that 50% or more, preferably 50% to 80%, and more preferably approximately 60% to 80% of blood flow can be ensured, for example. The number of sites of ligation is not particularly limited. Preferably, ligation is performed at 2 or more sites at appropriate intervals such as those of approximately a few millimeters to those of several dozen millimeters (and further preferably at approximately 5-mm to 50-mm intervals). As a surgical suture for rats, a No. 12 suture and the like can be used. Depending on animal types or artery types, a suture with an appropriate thickness can be selected. After arterial ligation, it is desired to suture the surgical sites, awaken the animal from anesthesia, and then provide about 1 to 7 days of a recovery period.

In this manner, a model animal of vascular wall lesion, which has a vascular wall lesion, can be produced with good reproducibility and a high success rate.

To evaluate vascular wall lesions in the present invention, a method employed therefor is not particularly limited. For example, when leukocyte and macrophage infiltration is confirmed in intravascular membranes along with confirmation of even a small number of blood platelet aggregates as a result of pathological observation using HE staining, this can be diagnosed as a vascular wall lesion.

Embodiments for use of the non-human model animal of vascular wall lesion of the present invention are not particularly limited. For example, the model animal can be suitably used for screening for medicines for diagnosis of vascular wall lesions and medicines for preventing and/or treating vascular wall lesions.

Test substances to be used herein are not particularly limited and any substances can be used. Examples of such test substances include peptides, proteins, non-peptidic compounds, synthetic low molecular weight compounds, fermentation products, cell extracts, plant extracts, and animal tissue extracts. These compounds may be novel compounds or known compounds. In addition, libraries containing many molecules, such as peptide libraries or compound libraries, can also be used as test substances.

The non-human model animal of vascular wall lesion of the present invention is characterized by a low mortality rate. Hence, the model animal can be efficiently used for screening for medicines for diagnosing vascular wall lesions and medicines for preventing and/or treating vascular wall lesions, and the like.

For example, a test substance is administered at approximately 0.01 mg/kg to 1000 mg/kg to the non-human model animal of vascular wall lesion of the present invention, and then the therapeutic effects of the test substance are examined using an effect of improving the vascular wall lesion, an effect of improving the mortality rate, and the like as indices. In this manner, preventive and/or therapeutic agent for the vascular wall lesion can be evaluated. Here, examples of treatment for vascular wall lesions include improvement of vascular wall lesions, suppression of the further development of vascular wall lesions, and prevention of increase in the severity of vascular wall lesions.

Furthermore, timing for administering a test substance to the non-human model animal of vascular wall lesion of the present invention may be a time before or after the development of a vascular lesion. Depending on the specific timing for administration, a drug for preventing or treating a vascular lesion can be evaluated.

A test substance that has been determined by the above screening to have an effect of improving a vascular wall lesion can be either orally or parenterally administered directly or in combination with pharmaceutically acceptable carriers. As a method for producing the test substance into a dosage form such as an oral formulation (e.g., tablets, pills, granules, powders, capsules, soft capsules, syrups, emulsions, and suspensions) or a parenteral formulation (e.g., injections and drops), any method known by persons skilled in the art can be employed. Furthermore, upon formulation, excipients, binders, disintegrants, lubricants, sweeteners, surfactants, suspending agents, emulsifiers, and the like can be appropriately combined therewith, if necessary.

The present invention will be further specifically described in the following examples. However, the examples are not intended to limit the scope of the present invention.

### Examples

### Example 1: Example of producing model rat of vascular wall lesion (arterial ligation method)

6-week-old male SD rats (produced by Charles River Laboratories Japan, Inc.) were purchased and then acclimatized for 1 week. During this week, rats with no abnormalities in terms of general symptoms were selected. 10 ml/kg Nembutal (produced by Banyu Pharmaceutical Co., Ltd.) was administered via the tail vein, so as to anesthetize each rat. The entire head portion was disinfected with rubbing alcohol (produced by Hikari Pharmaceutical Co., Ltd.). After disinfection, an incision about 2 cm long was made in the superficial epithelial portion of the skin at a location approximately 1 cm below the ear. The incised portion was disinfected again with the above rubbing alcohol. Next, the endothelial portion of the incised portion was incised. The length of the incision made at this time should be within 10 mm. A carotid artery was separated and removed from the vicinity of nerves and other blood vessels without damaging the artery. The thus obtained carotid artery was ligated at 2 positions at approximately 10-mm intervals using surgical sutures (No. 12 sutures produced by Natsume Seisakusho Co., Ltd.). At this time, the channel for blood flow was ligated to a maximum degree of approximately 50%. Blood flow must not be blocked to a level greater than such degree. After arterial ligation, the surgical sites were sutured (No. 10 sutures produced by Natsume Seisakusho Co., Ltd.).

After surgery, the rats were raised for 6 days with ORC-4 normal feed for rats (produced by Oriental Yeast Co., Ltd.), during which water was administered *ad libitum.*

As a result, pathological rats with vascular wall lesions could be produced with probability of 98% or higher (110 rats out of 112 rats), in which vascular wall lesions were formed at arterial ligation sites. The sites were diagnosed as vascular wall lesion, when leukocyte and macrophage infiltration was confirmed in intravascular membranes along with confirmation of even a small number of blood platelet aggregates as a result of pathological observation using HE staining.

### Comparative example 1: Balloon catheter method

6-week-old male SD rats (produced by Charles River Laboratories Japan, Inc.) were purchased and then acclimatized for 1 week. During this week, rats with no abnormalities in terms of general symptoms were selected. 10 ml/kg Nembutal (produced by Banyu Pharmaceutical Co., Ltd.) was administered via the tail vein, so as to anesthetize each rat. The entire head portion was disinfected with rubbing alcohol (produced by Hikari Pharmaceutical Co., Ltd.). After disinfection, an incision about 2 cm long was made in the superficial epithelial portion of the skin at a location approximately 1 cm below the ear. The incised portion was disinfected again with the above rubbing alcohol. Next, the inner membrane of the incised portion was incised. The length of the incision made at this time should be within 5 mm. After a carotid artery was removed, a balloon catheter (S-2962 produced by TERUMO Corporation, Japan) was inserted. The length of the tip to be inserted was 2 cm. Insertion was continued until the balloon catheter reached an obstruction. Air was sent to the tip portion to expand such portion and then air was removed from the tip portion for restoration. Bleeding at sites was stopped using gauze (produced by Hakujuji Co., Ltd.). After the bleeding had been stopped, the surgical sites were sutured (No. 10 sutures produced by Natsume Seisakusho Co., Ltd.).

After surgery, the rats were raised for 10 days with ORC-4 normal feed for rats (produced by Oriental Yeast Co., Ltd.), during which water was administered *ad libitum.*

As a result, 48% of all the rats developed vascular wall lesions (17 rats out of 36 rats). Among the remaining 52% (19 rats out of 36 rats) of the rats, 9 rats developed no vascular wall lesions and the remaining 10 rats died after surgery.

The sites were diagnosed as vascular wall lesion, when leukocyte and macrophage infiltration was confirmed in intravascular membranes along with confirmation of even a small number of blood platelet aggregates as a result of pathological observation using HE staining.

As revealed by the above results, the percentage of rats that formed vascular wall lesions was 98% in the case of the arterial ligation method, whereas the same in the case of the balloon catheter method was 48%. That is, the probability of forming pathological models in the case of the arterial ligation method was improved so that it became twice or more than that in the case of the balloon catheter method. Moreover, whereas the raising period in the case of the arterial ligation method was 6 days, the same in the case of the balloon catheter method was 10 days. The raising period in the case of the arterial ligation method of the present invention was significantly shorter than that of the balloon catheter method, by 4 days.

### Industrial Applicability

According to the method of the present invention, it is made possible to efficiently cause the development of a vascular wall lesion by invasion from outside the blood vessel and evaluate the diagnostic and therapeutic effects of a drug on the vascular lesion site using the animal having the lesion. The method of the present invention is a very safe method (e.g., low mortality rate of animals) and enables a shorter raising period as compared with those of conventional cases.

## Claims

1. A method for producing a non-human model animal of vascular wall lesion, which comprises invading a part of an artery of a non-human animal from outside the blood vessel and producing an animal having a lesion at the invaded vascular wall.

2. The method according to claim 1 wherein a part of an artery is invaded from outside the blood vessel by ligation of the blood vessel.

3. The method according to claim 2 wherein a part of an artery is invaded from outside the blood vessel by ligation of the blood vessel that enables ensuring a 50% to 80% blood flow.

4. The method according to claim 2 wherein ligation is performed at 2 or more sites at 5-mm to 50-mm intervals.

5. The method according to claim 1 wherein the animal is a dog, a miniature pig, a rabbit, a rat, a guinea pig, or a mouse.

6. The method according to claim 1 wherein the site of a vascular wall lesion is a carotid artery.

7. A method for evaluating a test substance, which comprises administering a test substance to a non-human model animal of vascular wall lesion which is produced by the method of claim 1, and evaluating the effects of the test substance on the vascular wall lesion.
